(19) Europäisches Patentamt / European Patent Office / Office européen des brevets

(11) **EP 4 335 520 A1**

(12) **EUROPEAN PATENT APPLICATION**
published in accordance with Art. 153(4) EPC

(43) Date of publication:
**13.03.2024 Bulletin 2024/11**

(21) Application number: **21941301.0**

(22) Date of filing: **12.05.2021**

(51) International Patent Classification (IPC):
**A63B 24/00** $^{(2006.01)}$

(52) Cooperative Patent Classification (CPC):
**A63B 24/00**

(86) International application number:
**PCT/CN2021/093238**

(87) International publication number:
**WO 2022/236726 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Guangdong Coros Sports Technology Joint Stock Company**
**Dongguan, Guangdong 523808 (CN)**

(72) Inventor: **LIU, Xin**
**Dongguan, Guangdong 523808 (CN)**

(74) Representative: **Rapisardi, Mariacristina**
**Ufficio Brevetti Rapisardi S.r.l.**
**Via Serbelloni, 12**
**20122 Milano (IT)**

(54) **METHOD AND APPARATUS FOR EVALUATING EXERCISE CAPABILITIES, DEVICE, AND STORAGE MEDIUM**

(57) Disclosed are a method and apparatus for evaluating an exercise capability, a device and a storage medium. The method for evaluating the exercise capability includes that: a target model equation pre-constructed for a user is invoked in response to an evaluation instruction for an exercise capability of the user, where the target model equation reflects a relationship between user paces and exercise time; and the exercise capability of the user is evaluated based on at least one user pace, where in the target model equation each of the at least one user pace corresponds to an exercise time point adapted in one of at least one exercise dimension.

In response to an evaluation instruction for an exercise capability of a user, invoke a target model equation pre-constructed for the user , where the target model equation reflects a relationship between user paces and exercise time — S 110

Evaluate the exercise capability of the user based on at least one user pace, where in the target model equation each of the at least one user pace corresponds to an exercise time point adapted in one of at least one exercise dimension — S 120

**FIG. 1**

**Description**

TECHNICAL FIELD

The present application relates to the technical field of exercise data monitoring, for example, to a method and apparatus for evaluating an exercise capability, a device and a storage medium.

BACKGROUND

Since running exercise is not restricted by factors such as fields and equipment, more and more users exercise through the running exercise. If the running pace of the user is too fast, then the user may not insist on running the whole running process, and if the running pace of the user is too slow, then the running performance of the user is affected. Therefore, the running pace during the exercise needs to be reasonably formulated according to the physical condition of the user, so as to achieve the reasonable exercise planning of the user during running.

For the evaluation of the physical strength, the evaluator generally performs a qualitative empirical analysis of the overall running capability of the runner according to the historical running condition of the runner. For some amateur runners, it is difficult to achieve the evaluation manner of the physical strength described above.

SUMMARY

The present application provides a method and apparatus for evaluating an exercise capability, a device and a storage medium, so as to implement the accurate evaluation of the exercise capability of the user and facilitate the reasonable planning of the user exercise.

A method for evaluating an exercise capability is provided. The method includes that: a target model equation pre-constructed for a user is invoked in response to an evaluation instruction for an exercise capability of the user, where the target model equation reflects a relationship between user paces and exercise time; and the exercise capability of the user is evaluated based on at least one user pace, where in the target model equation each of the at least one user pace corresponds to an exercise time point adapted in one of at least one exercise dimension.

There is further provided an apparatus for evaluating an exercise capability. The apparatus includes a model invocation module and an exercise capability evaluation module. The model invocation module is configured to invoke, in response to an evaluation instruction for an exercise capability of a user, a target model equation pre-constructed for a user, where the target model equation reflects a relationship between user paces and exercise time. The exercise capability evaluation module is configured to evaluate the exercise capability of the user based on at least one user pace, where in the target model equation each of the at least one user pace corresponds to an exercise time point adapted in at least one exercise dimension.

There is further provided an electronic device. The electronic device includes one or more processors and a storage apparatus. The storage apparatus is configured to store one or more programs. The one or more programs, when executed by the one or more processors, cause the one or more processors to implement the method for evaluating the exercise capability described above.

There is further provided a computer-readable storage medium. The computer-readable storage medium stores a computer program. The program, when executed by a processor, implements the method for evaluating the exercise capability described above.

BRIEF DESCRIPTION OF DRAWINGS

FIG. 1 is a flowchart of a method for evaluating an exercise capability according to an embodiment one of the present application;

FIG. 2 is a flowchart of a method for evaluating an exercise capability according to an embodiment two of the present application;

FIG. 3 is a schematic structural diagram of an apparatus for evaluating an exercise capability according to an embodiment three of the present application; and

FIG. 4 is a schematic structural diagram of an electronic device according to an embodiment four of the present application.

DETAILED DESCRIPTION

The present application will be described below in conjunction with the accompanying drawings and embodiments.

Embodiment one

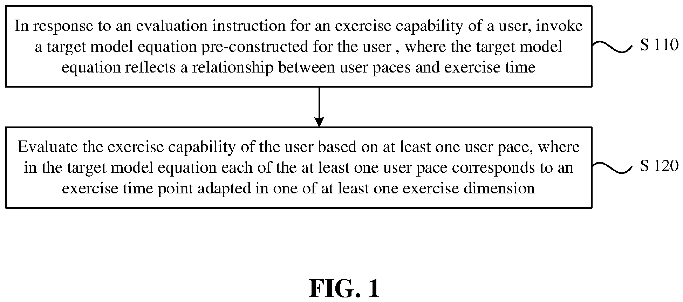

FIG. 1 is a flowchart of a method for evaluating an exercise capability according to an embodiment one of the present application. This embodiment may be applicable in any one of cases of evaluating user exercise capabilities. The method for evaluating an exercise capability provided in this embodiment may be performed by an apparatus for evaluating an exercise capability provided in the embodiments of the present application, and this apparatus may be implemented by means of software and/or hardware and may be integrated in an electronic device for performing the method.

Referring to FIG. 1, the method includes the following steps.

In S 110, in response to an evaluation instruction for an exercise capability of a user, a target model equation pre-constructed for the user is invoked, where the target model equation reflects a relationship between user paces and exercise time.

Optionally, since the exercise capability of the user is mainly divided into six exercise dimensions of explosive power, velocity, velocity endurance, aerobic, endurance, and super endurance, the six exercise dimensions may be represented by the user pace at different exercise time points, and the fast and slow of the user pace may also reflect the strength of the exercise capability of the user. On this basis, in this embodiment, exercise capabilities of the user in different exercise dimensions may be analyzed by analyzing paces of the user at different exercise time points. Therefore, the target model equation reflecting the relationship between user paces and exercise time can be constructed for the user to analyze the paces of the user at different exercise time points, thereby determining the exercise capability of the user.

When the evaluation instruction of the exercise capability of the user is detected, the target model equation pre-constructed for the user is firstly invoked, and the target model equation can reflect the relationship between user paces and exercise time, so as to subsequently analyze the paces of the user at different exercise time points, thereby determining the exercise capability of the user.

Exemplarily, the target model equation in this embodiment may be constructed in such a manner that the pace corresponding to the maximum oxygen uptake of the user is determined based on the standard pace of the user in the standard course; and the target model equation reflecting the relationship between user paces and exercise time is constructed by using the pace corresponding to the maximum oxygen uptake and the real-time pace in the actual exercise of the user.

Optionally, when the exercise capability of the user is analyzed, the maximum exercise strength that the user can withstand is mainly analyzed. Considering that the maximum oxygen uptake is the oxygen content ingested by the human body during vigorous exercise, it is possible to effectively reflect the aerobic exercise capability of the human body, and the user pace during exercise is the faster, then the exercise capability will be the stronger. Therefore, in this embodiment, the pace corresponding to the maximum oxygen uptake may be adopted to analyze the exercise capability of the user.

In this embodiment, since the pace corresponding to the maximum oxygen uptake is a pace that the user can achieve during strenuous exercise, it is possible to determine the pace adopted when the user supports running through the whole course at the maximum exercise intensity by analyzing the exercise situation of the user during the actual exercise, that is, the standard pace of the user in the standard course is calculated, and then the pace corresponding to the maximum oxygen uptake of the user is determined by using the standard pace and an actual oxygen uptake situation of the user in the standard course, where the standard course in this embodiment may be divided into a half marathon and a full marathon and the like.

As an alternative to this embodiment, the calculation process of the pace corresponding to the maximum oxygen uptake may include: a lactate threshold pace corresponding to the standard pace is determined by using the standard pace of the user in the standard course; the pace corresponding to the maximum oxygen uptake of the user is determined based on the lactate threshold pace.

For example, the standard course is a half marathon, as shown in Table 1 below, multiple items of data of the user during the historical exercise of the standard course are collected in advance to form a matching data table between the standard pace, the lactate threshold pace and the pace corresponding to the maximum oxygen uptake.

Table 1 Matching data table between standard pace, lactate threshold pace, and pace corresponding to the maximum oxygen uptake at half-course

| Standard pace | Lactate threshold pace | Pace corresponding to the maximum oxygen uptake |
| --- | --- | --- |
| 2:45 | 2:44 | 2:44/120% |
| 3:25 | 3:24 | 3:24/120% |
| 4:05 | 4:02 | 4:02/120% |
| 4:47 | 4:40 | 4:40/118% |
| 5:30 | 5:16 | 5:16/117% |
| 6:13 | 6:03 | 6:03/115% |
| 6:52 | 6:27 | 6:27/114% |
| 7:25 | 7:15 | 7:15/113% |
| 8:03 | 7:40 | 7:40/112% |
| 8:55 | 8:20 | 8:20/111% |
| 9:29 | 9:10 | 9:10/110% |

In different standard courses, a standard pace, a lactate threshold pace and a pace corresponding to the maximum oxygen uptake and corresponding to each of the different standard courses may be obtained. According to the standard pace, the lactate threshold pace and the pace corresponding to the maximum oxygen uptake and corresponding to the each of the different standard courses, a first correlation equation reflecting a relationship between the standard pace and the lactate threshold pace and a second correlation equation reflecting a relationship between the lactate threshold pace and the pace corresponding to the maximum oxygen uptake may be fitted in the standard courses. Therefore, the lactate threshold pace corresponding to a standard pace may be obtained by inputting the standard pace of the user in a standard course into the first correlation equation, and then the pace corresponding to the maximum oxygen uptake and corresponding to the lactate threshold pace may be obtained by inputting the lactate threshold pace into the second correlation equation, that is, the pace corresponding to the maximum oxygen uptake of the user in this embodiment.

In this embodiment, the abscissa of the target model equation may represent a change in exercise time, and the ordinate of the target model equation may represent a change in the user pace.

Since the real-time pace of the user is correspondingly reduced along with the increase of the exercise time in the actual exercise process of the user, so that the paces of the user at different exercise time points may be preliminarily analyzed by referring to the pace corresponding to the maximum oxygen uptake of the user, and then whether the preliminarily analyzed pace of the user at different exercise time points satisfies the actual exercise is determined and correspondingly adjusted by adopting the real-time paces of the user at different exercise time points in the actual exercise of the user, so that a large number of data points satisfying the actual exercise of the user at different exercise time points are obtained, and a corresponding target model equation may be fitted through the data points to accurately reflect the relationship between user paces and exercise time.

In S120, the exercise capability of the user is evaluated based on at least one user pace, where in the target model equation each of the at least one user pace corresponds to an exercise time point adapted in one of at least one exercise dimension.

Optionally, since the explosive power, velocity, velocity endurance, aerobic, endurance, and super endurance in the exercise dimension are respectively represented by paces of the user at different exercise time points, the exercise time point indicated by each exercise dimension is input to the established target model equation to obtain user paces of the user at each exercise dimension, and then the fast and slow of the user pace at each exercise dimension is analyzed so as to evaluate the overall exercise capability of the user, thereby achieving the accurate evaluation of the exercise capability of the user.

As an alternative to this embodiment, that the exercise capability of the user is evaluated based on the at least one user pace, where in the target model equation each of the at least one user pace corresponds to the exercise time point adapted in one of the at least one exercise dimension includes that: a user pace in the each exercise dimension in the target model equation is determined based on an exercise time point adapted in each exercise dimension of the at least one exercise dimension; an exercise capability score of the user in the each exercise dimension is determined based on the user pace in the each exercise dimension and a preset exercise capability score lookup table in the each exercise dimension; and a comprehensive exercise capability of the user is evaluated based on at least one exercise capability score of the user in the at least one exercise dimension.

The exercise time point adapted in each exercise dimension is determined, for example, an exercise time point adapted for the explosive power is the 10th second in the target model equation, an exercise time point adapted for the velocity is the 60th second in the target model equation, an exercise time point adapted for the velocity endurance is the 5th minute in the target model equation, an exercise time point adapted for the aerobic is the 60th minute in the target model equation, an exercise time point adapted for the endurance is the 120th minute in the target model equation, and an exercise time point adapted for the super endurance is the 600th minute in the target model equation. Exercise time points adapted in each exercise dimension are respectively input into the constructed target model equation, so that the user pace of the user in each exercise dimension may be determined.

In order to analyze the exercise capability score in each exercise dimension, in this embodiment, a lookup table of a relationship between the user pace and the exercise capability score is set in advance in each exercise dimension, and the exercise capability score corresponding to the user pace of the user in the exercise dimension is determined by using the user pace in the exercise dimension and the exercise capability score lookup table preset in this exercise dimension.

Exemplarily, the exercise capability score lookup table in each exercise dimension is shown in Tables 2 to 7.

Table 2 Explosive power score

| User pace (minute/km) | Explosive power score |
|---|---|
| 1:35 | 100 |
| 1:50 | 80 |
| 2:00 | 70 |
| 2:10 | 60 |
| 2:30 | 40 |
| 3:20 | 0 |

Table 3 Velocity score

| User pace (minute/km) | Velocity score |
|---|---|
| 1:50 | 100 |
| 2:30 | 80 |
| 2:42 | 70 |
| 3:00 | 60 |
| 3:20 | 40 |
| 5:00 | 0 |

Table 4 Aerobic score

| User pace (minute/km) | Aerobic score |
|---|---|
| 2:45 | 100 |
| 3:00 | 80 |
| 3:30 | 70 |
| 4:30 | 60 |
| 6:00 | 40 |
| 9:00 | 0 |

Table 5 Velocity endurance score

| User pace (minute/km) | Velocity endurance score |
|---|---|
| 2:00 | 100 |
| 2:40 | 80 |
| 3:20 | 70 |
| 3:50 | 60 |
| 4:30 | 40 |
| 7:00 | 0 |

Table 6 Endurance score

| User pace (minute/km) | Endurance score |
|---|---|
| 2:50 | 100 |
| 3:20 | 80 |
| 4:00 | 70 |
| 4:30 | 60 |
| 5:30 | 40 |
| 8:00 | 0 |

Table 7 Super endurance score

| User pace (minute/km) | Super endurance score |
|---|---|
| 3:40 | 100 |
| 4:30 | 80 |
| 5:30 | 70 |
| 7:00 | 60 |
| 8:00 | 40 |
| 20:00 | 0 |

From the above tables, the exercise capability score of the user in each exercise dimension may be determined according to the user pace in the each exercise dimension. Then, an average value of the scores of the user in the multiple exercise capability scores is used as the comprehensive exercise capability of the user. The exercise capability of the user may be divided into five grades of leisure (0-40), general (41-60), good (60-70), excellent (70-80) and cyanine (80-100), and grade of the exercise capability of the user may be evaluated according to the comprehensive ability score of the user. According to grade of the exercise capability of the user, a reasonable exercise plan may be made for the user to ensure the health management of the user exercise.

According to the technical scheme provided in this embodiment, in response to the evaluation instruction for the exercise capability of the user, the target model equation pre-constructed for the user is firstly invoked, and the target model equation can reflect the relationship between user paces and exercise time. Then, considering that paces of the user at different exercise time points can reflect exercise capabilities of the user at different dimensions, the exercise capability of the user is evaluated based on a user pace corresponding to an exercise time point in the target model equation and adapted in each exercise dimension, so that the automatic evaluation of the exercise capability of the user is achieved without subjective qualitative empirical analysis by an evaluator, and the evaluation accuracy of the exercise capability is improved, whereby a reasonable exercise planning may be made according to the exercise capability of the user, and thus the health management of the user exercise can be ensured.

Embodiment two

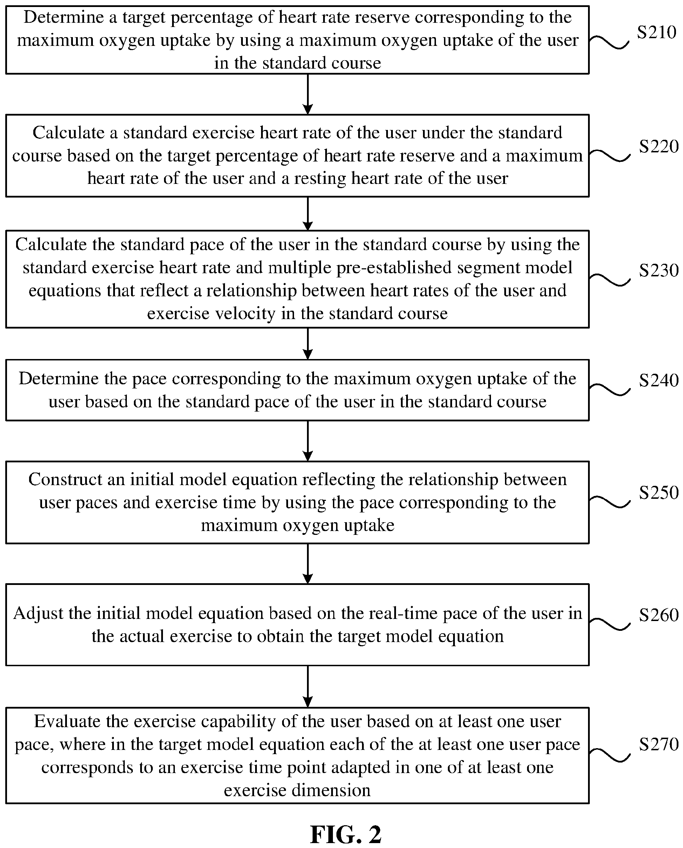

FIG. 2 is a flowchart of a method for evaluating an exercise capability according to an embodiment two of the present application. The embodiment of the present application is described on the basis of the above-described embodiments. Optionally, the calculation process of the standard pace of the user in the standard course and the construction process of the target model equation are mainly described in this embodiment.

Referring to FIG. 2, the method of this embodiment may include steps described below.

In S210, a target percentage of heart rate reserve corresponding to the maximum oxygen uptake is determined by using a maximum oxygen uptake of the user in the standard course.

According to this embodiment, the maximum oxygen uptake supported by the user in the standard course may be analyzed through the physiological characteristics such as a user age, a user sex, and a user weight, the maximum heart rate and the resting heart rate of the user in the standard course, and the average exercise heart rate and the average exercise velocity calculated by the real-time exercise heart rate and the real-time exercise velocity of the user in the next period of exercise in the standard course, and the maximum oxygen uptake in this embodiment can be calculated as: $V_{O_2 max} = A + P_1 * S - P_2 * G + P_3 * V * \dfrac{P_4}{B} - C * \dfrac{hr_{avg} - hr_0}{hr_{max} - hr_0} - \dfrac{2(a-26)}{5}$ ; where $A$ is a constant of 40 to 50, $P_1$ is a constant of 7 to 8, $S$ is a sex constant, 1 is for males, and 0 is for females; $P_2$ is a constant of 0.1 to 0.2 and $G$ is the weight of the user; $P_3$ is a constant of 4 to 5, $V$ is an average exercise velocity, $P_4$ is a constant of 3 to 4, and $B$ is a constant of 1 to 2; $C$ is a constant of 15 to 20, $hr_{avg}$ is an average exercise heart rate, $hr_0$ is a resting heart rate of the user in the awake and quiet state, and $hr_{max}$ is a maximum heart rate of the user; and $a$ is the user age.

A percentage of heart rate reserve lookup table corresponding to the standard course may be made according to the actual exercise of the user in the standard course. The half marathon is used as an example, as shown in Table 8 and Table 9 below, and percentages of heart rate reserve corresponding to users with different physiological characteristics and maximum oxygen uptake relative to the half marathon are recorded in the user percentage of heart rate reserve lookup table.

Table 8 Percentage of heart rate reserve of men at half marathon

| | 75% | 78% (may be any percentage between 75% and 80%) | 80% |
|---|---|---|---|
| Age (male) | Maximum oxygen uptake region 1 | Maximum oxygen uptake region 2 | Maximum oxygen uptake region 3 |
| 29 and below | 45 and below | 46-59 | 60 and above |
| 30-34 | 43 and below | 44-57 | 58 and above |
| 35-39 | 41 and below | 42-55 | 56 and above |
| 40-44 | 39 and below | 40-53 | 54 and above |
| 45-49 | 37 and below | 38-51 | 52 and above |
| 50-54 | 35 and below | 36-49 | 50 and above |
| 55-59 | 33 and below | 34-47 | 48 and above |
| 60-64 | 31 and below | 32-45 | 46 and above |
| 65 and above | 29 and below | 30-43 | 43 and above |

Table 9 Percentage of heart rate reserve of women at half marathon

| | 75% | 78% (may be any percentage between 75% and 80%) | 80% |
|---|---|---|---|
| Age (female) | Maximum oxygen uptake region 1 | Maximum oxygen uptake region 2 | Maximum oxygen uptake region 3 |
| 29 and below | 38 and below | 39-52 | 53 and above |
| 30-34 | 36 and below | 37-50 | 51 and above |
| 35-39 | 34 and below | 35-48 | 49 and above |
| 40-44 | 32 and below | 33-46 | 47 and above |
| 45-49 | 30 and below | 31-44 | 45 and above |
| 50-54 | 28 and below | 29-42 | 43 and above |
| 55-59 | 26 and below | 27-40 | 41 and above |
| 60-64 | 24 and below | 25-38 | 39 and above |
| 65 and above | 22 and below | 23-36 | 37 and above |

The percentage of heart rate reserve lookup table corresponding to the above-described half marathon may be made for different standard courses. The target percentage of heart rate reserve of the user relative to the standard course may be obtained by querying the percentage of heart rate reserve under the standard course based on the physiological characteristic data of the user and the maximum oxygen uptake.

In S220, a standard exercise heart rate of the user under the standard course is calculated based on the target percentage of heart rate reserve and a maximum heart rate of the user and a resting heart rate of the user.

The percentage of heart rate reserve is calculated as: $\rho = \dfrac{hr_i - hr_0}{hr_{max} - hr_0}$, where $hr_i$ is the real-time heart rate of the user during exercise, $hr_{max}$ is the maximum heart rate of the user, and $hr_0$ is the resting heart rate of the user in the awake and quiet state. The standard exercise heart rate of the user in the standard course may be calculated by substituting the target percentage of heart rate reserve of the user relative to the standard course and the maximum heart rate and the resting heart rate of the user into the above-described calculation formula.

In this embodiment, the maximum heart rate and the resting heart rate of the user may be detected by the portable heart rate detection device, and the user may also manually set when the user knows the maximum heart rate value and the resting heart rate value of the user. In addition, the maximum heart rate may also be obtained by calculating the user age, that is, $hr_{max} = 208 - 0.7a$, and $a$ is the user age.

In S230, the standard pace of the user in the standard course is calculated by using the standard exercise heart rate and multiple pre-established segment model equations that reflect a relationship between heart rates of the user and exercise velocity in the standard course.

In order to calculate the standard pace of the user in the standard course by using the standard exercise heart rate, a large number of effective historical heart rates and historical exercise velocities need to be selected to establish a model equation reflecting the relationship between heart rates of the user and the exercise velocities in the standard course. However, since the standard course is divided into different courses such as a half marathon and a full marathon, in order to calculate the standard pace in each standard course, in this embodiment, the collected data of multiple types are divided into five segments of 0 km - 10 km, 10 km - 20 km, 20 km - 30 km, 30 km - 40 km and more than 40 km according to the exercise distance, and a corresponding segment model equation may be established in each segment, so that different standard courses may be obtained according to the multiple segments, and standard paces in different standard courses may be calculated according to the corresponding exercise velocities.

Exemplarily, in this embodiment, the real-time heart rate, the pace, the temperature, the humidity, the altitude, the gradient, the distance, the body temperature, the exercise time and other data of the user in the historical exercise process may be acquired, the heart rate and pace data that match the model construction are selected, and the requirements are as follows.

1) The duration of a single continuous exercise of the user reaches a preset duration (such as 10 minutes); 2) the historical heart rate and the historical exercise velocity in each continuous unit time period is sequentially acquired in a certain moving period and a time sequence, and whether the historical heart rate and the historical exercise velocity in each continuous unit time period satisfy the following conditions is sequentially determined: a, and the heart rate fluctuation in the unit time period is within a first preset range (not exceeding 12bpm); b. the velocity fluctuation within the unit time period is within a second preset range (not exceeding 5m/s); c. the real-time percentage of heart rate reserve within the unit time period is within a third preset range (50%~90%). That is, in a case where the continuous exercise duration of the user reaches a preset duration, the continuous exercise duration is divided into multiple unit time periods, there is a period overlap between adjacent unit time periods, and a time difference between start time points corresponding to adjacent unit time periods is one exercise period, and it is determined whether the historical heart rate and the historical pace within each unit time period satisfy the above conditions. It is possible to continuously acquire the historical heart rate and the historical exercise velocity within each target unit time period satisfying the above-described conditions, and calculate an average value of the historical heart rate and an average value of the historical exercise velocity within each target unit time period as corresponding associated data points. For example, one unit time period is 2 minutes, the heart rate and the exercise velocity within each 2 minutes are continuously recorded in a moving period of 1 second, and an average value of the heart rate and an average value of the exercise velocity within each 2 minutes is calculated, that is, the unit time period of one 2 minutes is counted every 1 second, so as to continuously acquire multiple data such as historical heart rate and historical exercise velocity within multiple continuous unit time periods such as 1-120 seconds, 2-121 seconds, 3-122 seconds, ...... and the like.

In this embodiment, the heart rate and the pace data points satisfying the above-described conditions may be classified according to exercise time, temperature, humidity, altitude, distance segment, body temperature, and slope. The exercise time may be divided into morning (5:00-7:00), morning (7:00-11:30), noon (11:30-14:30), afternoon (14:30-17:00), evening (17:00-19:00), evening (19:00-22:00) and night (22:00-5:00). The temperature may be divided into -10 degrees below, -10-0 degrees, 0-10 degrees, 10-20 degrees, 20-25 degrees, 25-30 degrees and more than 30 degrees. The humidity may be divided into 30 percent below, 30 percent to 60 percent, 60 percent to 80 percent, 80 percent to 90 percent and more than 90 percent. The altitude may be divided into 500 m below, 500 m - 1000 m, 1000 m - 1500 m, 1500 m - 2500 m, 2500 m - 3500m, 3500 m - 4000m, 4000 m - 5000 m and more than 5000 m. The distance segment may be divided into 0 km - 10 km, 10 km - 20 km, 20 km - 30 km, 30 km - 40 km and more than 40 km. The body temperature may be divided into 36 degrees below, 36-37 degrees, 37-38 degrees, 38-39 degrees and more than 39 degrees. The gradient may be divided into -45 degrees below, -45 degrees to -30 degrees, -30 degrees to 20 degrees, -20 degrees to 10 degrees, -10 degrees to 0 degrees, 0 degrees to 5 degrees, 5 degrees to 10 degrees, 10 degrees to 20 degrees, 20 degrees to 30 degrees, 30 degrees to 40 degrees, 40 degrees to 50 degrees and more than 50 degrees.

According to the multiple exercise distances, the associated data points composed of the historical heart rate and the historical exercise velocity within the multiple target unit time periods are classified according to distance segment to form the corresponding data sets under the five segments of 0 km - 10km, 10 km - 20 km, 20 km - 30 km, 30 km - 40 km, and more than 40 km, so that the segment model equation for reflecting the relationship between heart rates of the user and the exercise velocities under each segment may be fitted by using the data sets under the each segment. For example, the segment model equation under each segment may be: $v_i = \dfrac{hr_i - B}{A}$ . The equation is used for calculating the corresponding exercise velocity by utilizing the standard exercise heart rate of the user in the standard course, and then calculating the corresponding standard pace by utilizing this exercise velocity. Or, in order to ensure the accuracy of the standard pace, the segment model equation may also be:

$$v_i = \frac{hr_i + time*C + atmos*D + humi*E + hei*F + slope*G + temp*H - B}{A}$$

. Where, *time* is the exercise time point, *atmos* is the air temperature, *humi* is the humidity, *hei* is the altitude, *slope* is the slope, *temp* is the body temperature, and *A, B, C, D, E, F,* G, and *H* are all constants. The accuracy of the multiple segment model equations is ensured by adding influence parameters.

Therefore, based on the above-described historical data analysis, five segment model equations for reflecting the relationship between the heart rate and the exercise velocity of the user under the five segments of 0 km - 10 km, 10 km - 20 km, 20 km - 30 km, 30 km - 40 km, more than 40 km and the like may be established, the segment model equation related to the standard course may be selected from the five segment model equations, then the standard exercise heart rate of the user under the standard course is input into the segment model equation related to the standard course so as to calculate a corresponding exercise velocity, and further the corresponding standard pace is calculated according to the exercise velocity in the segment model equation.

The half marathon is used as an example of the standard course, it is determined that the segment model equations under 0 km - 10 km and 10 km - 20 km are related to the half marathon, the standard exercise heart rates of the user under the half marathon are respectively input into the segment model equations under 0 km - 10 km and 10 km - 20 km, a first exercise velocity $v_1$ under 0 km - 10 km and a second exercise velocity $v_2$ under 10 km - 20 km may be obtained, respectively, and the half marathon pace is calculated to be $\dfrac{10/v_1 + 10/v_2}{20}$ .

Referring to the above-described steps, the standard pace of the user in the standard course may be calculated through multiple segment model equations and the standard exercise heart rate in the standard course, so that the corresponding lactate threshold pace may be determined by adopting the standard pace in the standard course in the following process, and the pace corresponding to the maximum oxygen uptake of the user may be determined based on the lactate threshold pace.

In S240, the pace corresponding to the maximum oxygen uptake of the user is determined based on the standard pace of the user in the standard course.

In S250, an initial model equation reflecting the relationship between user paces and exercise time is constructed by using the pace corresponding to the maximum oxygen uptake.

Optionally, due to in the actual exercise of the user, the user pace may gradually decrease with the increase of the exercise time, so that in this embodiment, user initial paces of the user at different exercise time points may be preliminarily calculated by using multiple percentage ratios of the pace corresponding to the maximum oxygen uptake of the user relative to different exercise time points, so that multiple exercise time points and the user initial paces at the multiple exercise time points are combined to obtain a large number of data points, and then, an initial model equation that reflects the relationship between the user paces and the exercise time may be fitted by using the multiple data points.

Exemplarily, the user initial paces at different exercise time points of the user may be that: a user pace corresponding to the exercise time point of 10s is a 120% the pace corresponding to the maximum oxygen uptake, a user pace corresponding to the exercise time point of 1 min is a 115% the pace corresponding to the maximum oxygen uptake, and a user pace corresponding to the exercise time point of 3 min is a 110% the pace corresponding to the maximum oxygen uptake, a user pace corresponding to the exercise time point of 5 min is 105% the pace corresponding to the maximum oxygen uptake, and a user pace corresponding to the exercise time point of 10 min is 100% the pace corresponding to the maximum oxygen uptake, a user pace corresponding to the exercise time point of 30 min is 95% the pace corresponding to the maximum oxygen uptake, while a user pace corresponding to the exercise time point of 90 min is 85% the pace corresponding to the maximum oxygen uptake, a user pace corresponding to the exercise time point of 180 min is 80% the pace corresponding to the maximum oxygen uptake, and a user pace corresponding to the exercise time point of 240 min is 75% the pace corresponding to the maximum oxygen uptake, a user pace corresponding to the exercise time point of 1200 min is a 60% the pace corresponding to the maximum oxygen uptake, and while a user pace corresponding to the exercise time point of more than 1200 min is a 60% the pace corresponding to the maximum oxygen uptake.

In S260, the initial model equation is adjusted based on the real-time pace of the user in the actual exercise to obtain the target model equation.

Optionally, in order to ensure the accuracy of the target model equation, after the initial model equation is constructed, the real-time paces of the user at different exercise time points in the actual exercise may be acquired, and the initial model equation may be adjusted correspondingly by comparing the real-time pace at each of the different exercise time points with the pace at the each of the different exercise time points in the initial model equation, so that the change of the pace related to the exercise time in the initial model equation may be constantly close to the actual exercise of the user, thereby obtaining the corresponding target model equation.

As an alternative to this embodiment, when the initial model equation is adjusted, a difference between the real-time pace of the user at each exercise time point in the actual exercise and the estimated pace at the each exercise time point in the initial model equation is determined, and the initial model equation is adjusted based on this difference. That is, if a real-time pace of the user at an exercise time point in the actual exercise is higher than an estimated pace at the exercise time point in the initial model equation, then the user pace in the vicinity of the exercise time point in the initial model equation is increased, and if a real-time pace of the user at an exercise time point in the actual exercise is lower than an estimated pace at the exercise time point in the initial model equation, then the user pace in the vicinity of the exercise time point in the initial model equation is decreased so that the user pace at multiple exercise time points in the adjusted initial model equation can be close to the actual exercise of the user.

In S270, the exercise capability of the user is evaluated based on at least one user pace, where in the target model equation each of the at least one user pace corresponds to an exercise time point adapted in one of at least one exercise dimension.

According to the technical schemes provided in this embodiment, the pace corresponding to the maximum oxygen uptake of the user is determined based on the standard pace of the user in the standard course, and then the target model equation reflecting the relationship between user paces and exercise time is constructed by using the pace corresponding to the maximum oxygen uptake and the real-time pace of the user in the actual exercise, considering that paces of the user at different exercise time points can reflect exercise capabilities of the user at different dimensions, the exercise capability of the user is evaluated based on a user pace corresponding to an exercise time point in the target model equation and adapted in each exercise dimension, so that the automatic evaluation of the exercise capability of the user is achieved without subjective qualitative empirical analysis by an evaluator, and the evaluation accuracy of the exercise capability is improved, whereby a reasonable exercise planning may be made according to the exercise capability of the user, and thus the health management of the user exercise can be ensured.

Embodiment three

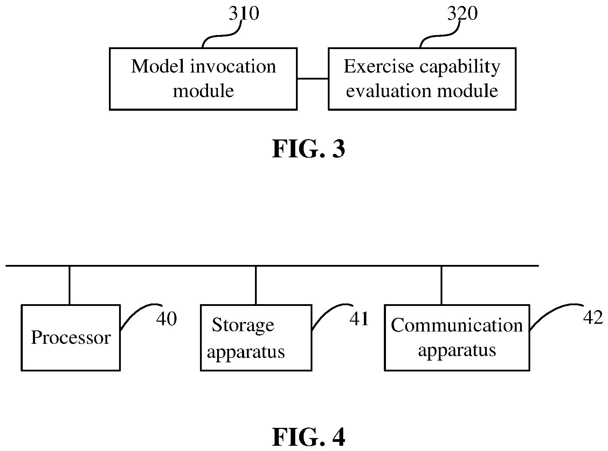

FIG. 3 is a schematic structural diagram of an apparatus for evaluating an exercise capability according to an embodiment three of the present application. As shown in FIG. 3, the apparatus may include a model invocation module 310 and an exercise capability evaluation module 320. The model invocation module 310 is configured to invoke, in response to an evaluation instruction for an exercise capability of a user, a target model equation pre-constructed for the user, where the target model equation reflects a relationship between user paces and exercise time. The exercise capability evaluation module 320 is configured to evaluate the exercise capability of the user based on at least one user pace, where in the target model equation each of the at least one user pace corresponds to an exercise time point adapted in one of at least one exercise dimension.

According to the technical scheme provided in this embodiment, in response to the evaluation instruction for the exercise capability of the user, the target model equation pre-constructed for the user is firstly invoked, and the target model equation can reflect the relationship between user paces and exercise time. Then, considering that paces of the user at different exercise time points can reflect exercise capabilities of the user at different dimensions, the exercise capability of the user is evaluated based on a user pace corresponding to an exercise time point in the target model equation and adapted in each exercise dimension, so that the automatic evaluation of the exercise capability of the user is achieved without subjective qualitative empirical analysis by an evaluator, and the evaluation accuracy of the exercise capability is improved, whereby a reasonable exercise planning may be made according to the exercise capability of the user, and thus the health management of the user exercise can be ensured.

The apparatus for evaluating the exercise capability described above may further include an oxygen uptake pace determination module and a model equation construction module. The target model equation described above may be constructed using the oxygen uptake pace determination module and the model equation construction module described above.

The oxygen uptake pace determination module is configured to determine a pace corresponding to a maximum oxygen uptake of the user based on a standard pace of the user in a standard course. The model equation construction module is configured to construct the target model equation reflecting the relationship between user paces and exercise time by using the pace corresponding to the maximum oxygen uptake and the user real-time pace in the actual exercise.

The oxygen uptake pace determination module may be configured to: determine a lactate threshold pace corresponding to the standard pace by using the standard pace of the user in the standard course; and determine a pace corresponding to a maximum oxygen uptake of the user based on the lactate threshold pace.

The apparatus for evaluating the exercise capability may further include a heart rate ratio determination module, a standard heart rate calculation module, a standard pace calculation module. The heart rate ratio determination module is configured to determine a target percentage of heart rate reserve corresponding to the maximum oxygen uptake by a user in a standard course. The standard heart rate calculation module is configured to calculate a standard exercise heart rate of the user under the standard course based on the target percentage of heart rate reserve and a maximum heart rate of the user and a resting heart rate of the user. The standard pace calculation module configured to calculate, by using the standard exercise heart rate and multiple pre-established segment model equations that reflect a relationship between heart rates of the user and paces in the standard course, the standard pace of the user in the standard course.

The multiple segment model equations may be established by using the following steps:
in a case where a continuous exercise duration of the user reaches a preset duration, the continuous exercise duration is divided into multiple unit time periods, where time periods overlap between adjacent unit time periods, and a time difference between initial time points respectively corresponding to the adjacent unit time periods is an exercise period, it is determined that whether a historical heart rate and a historical pace within each unit time period of the multiple unit time periods satisfy following conditions: 1) a heart rate fluctuation within the each unit time period is within a first preset range; 2) a velocity fluctuation within the each unit time period is within a second preset range; and 3) a real-time percentage of heart rate reserve within the each unit time period is varying within a third preset range; an average value of historical heart rates and an average value of historical exercise velocities in each target unit time period which satisfies the above-described conditions is calculated to use as an associated data point reflecting a relationship between heart rates of the user and paces, and associated data points corresponding to multiple target unit time periods are classified according to multiple predetermined segment exercise distances; and a segment model equation under each segment exercise distance is fitted by using associated data points corresponding to multiple target unit time periods within the each segment exercise distance of the multiple segment exercise distances.

The above-described model equation construction module may include an initial model construction unit and a model adjustment unit. The initial model construction unit is configured to: construct, by using the pace corresponding to the maximum oxygen uptake, an initial model equation reflecting the relationship between user paces and exercise time. The model adjustment unit is configured to adjust, based on the real-time pace of the user in the actual exercise, the initial model equation to obtain the target model equation.

The model adjustment unit may be configured to: determine a difference between a real-time pace of the user at each exercise time point in the actual exercise and an estimated pace at the each exercise time point in the initial model equation, and adjust the initial model equation based on the difference.

The exercise capability evaluation module 320 may be configured to: determine, based on an exercise time point adapted in each exercise dimension of the at least one exercise dimension, in the target model equation, a user pace in the each exercise dimension; determine, based on the user pace in the each exercise dimension of the at least one exercise dimension and a preset exercise capability score lookup table in the each exercise dimension, an exercise capability score of the user in the each exercise dimension; and evaluate a comprehensive exercise capability of the user based on the exercise capability score of the user in the at least one exercise dimension.

The apparatus for evaluating the exercise capability provided in this embodiment is applicable to the method for evaluating the exercise capability provided in any of the above-described embodiments, and has corresponding functions and effects.

Embodiment four

FIG. 4 is a schematic structural diagram of an electronic device according to an embodiment four of the present application. As shown in FIG. 4, the electronic device includes a processor 40, a storage apparatus 41 and a communication apparatus 42. A number of processors 40 in the electronic device may be one or more, one processor 40 is used as an example in FIG. 4. The processor 40, the storage apparatus 41 and the communication apparatus 42 in the electronic device may be connected by a bus or otherwise, and they being connected by a bus is used as an example in FIG. 4.

As a computer-readable storage medium, the storage apparatus 41 may be provided to store a software program, a computer-executable program, and a module such as a module corresponding to the method for evaluating the exercise capability in the embodiments of the present application (such as, the model invocation module 310 and the exercise capability evaluation module 320 in the apparatus for evaluating the exercise capability). The processor 40 is configured to execute multiple functional applications and data processing of the electronic device by running a software program, an instruction, and a module stored in the storage apparatus 41, that is, the method for evaluating the exercise capability described above is achieved.

The storage apparatus 41 may mainly include a storage program region and a storage data region. The storage program region may store an operating system, and an application program required for at least one function. The storage data region may store data and the like created according to the use of the terminal. Moreover, the storage apparatus 41 may include a high-velocity random access memory and may also include a non-volatile memory such as at least one magnetic disk storage device, flash storage device, or other non-volatile solid-state storage devices. In some instances, the storage apparatus 41 may include a memory disposed remotely with respect to a multi-function controller 40, and these remote memories may be connected to the electronic device over a network. Instances of such networks include, but are not limited to, an Internet, an intranet, a local area network, a mobile communication network, and combinations thereof.

The communication apparatus 42 may be configured to implement a network connection or a mobile data connection between devices.

The electronic device provided in this embodiment may be configured to perform the method for evaluating the exercise capability provided in any of the embodiments described above, and the electronic device provided in this embodiment has corresponding functions and effects.

Embodiment five

An embodiment five of the present application further provides a computer-readable storage medium. The computer-readable storage medium stores a computer program. The program may, when executed by a processor, implement the method for evaluating the exercise capability in any of the embodiments described above. The method includes that: a target model equation pre-constructed for a user is invoked in response to an evaluation instruction for an exercise capability of the user, where the target model equation reflects a relationship between user paces and exercise time; and the exercise capability of the user is evaluated based on at least one user pace where in the target model equation each of the at least one user pace corresponds to an exercise time point adapted in at least one exercise dimension.

An embodiment of the present application provides a storage medium including a computer-executable instruction, the computer-executable instruction is not limited to the method operation described above, but may also perform related operations in the method for evaluating the exercise capability provided in any of the embodiments of the present application.

From the above description of the implementation manners, the present application may be implemented by means of software and necessary general purpose hardware, and may also be implemented by hardware. The technical schemes of the present application may essentially be embodied in the form of a software product, the computer software product may be stored in a computer readable storage medium, such as a floppy disk of a computer, a read-only memory (ROM), a random access memory (RAM), a flash memory (FLASH), a hard disk or an optional disk, and the computer software product includes multiple instructions to enable a computer device (which may be a personal computer, a server, or a network device, and the like) to perform the method described in the embodiments of the present application.

In the above-described embodiments of the apparatus for evaluating the exercise capability, multiple units and modules included in the apparatus for evaluating the exercise capability are only divided according to the function logic and are not limited to the above-described division, as long as corresponding functions may be achieved. In addition, the names of multiple functional units are also merely to facilitate distinguishing from each other and are not intended to limit the scope of protection of the present application.

**Claims**

1. A method for evaluating an exercise capability, comprising:

   invoking, in response to an evaluation instruction for an exercise capability of a user, a target model equation pre-constructed for the user, wherein the target model equation reflects a relationship between user paces and exercise time; and
   evaluating the exercise capability of the user based on at least one user pace, wherein in the target model equation each of the at least one user pace corresponds to an exercise time point adapted in one of at least one exercise dimension.

2. The method of claim 1, wherein the target model equation is constructed by adopting following steps:

   determining a pace corresponding to a maximum oxygen uptake of the user based on a standard pace of the user in a standard course; and
   constructing the target model equation by using the pace corresponding to the maximum oxygen uptake and a real-time pace of the user in an actual exercise.

3. The method of claim 2, wherein determining the pace corresponding to the maximum oxygen uptake of the user based on the standard pace of the user in the standard course comprises:

   determining a lactate threshold pace corresponding to the standard pace by using the standard pace of the user in the standard course; and
   determining the pace corresponding to the maximum oxygen uptake of the user based on the lactate threshold pace.

4. The method of claim 2, before determining the pace corresponding to the maximum oxygen uptake of the user based on the standard pace of the user in the standard course, the method further comprises:

   determining, by using a maximum oxygen uptake of the user in the standard course, a target percentage of heart rate reserve corresponding to the maximum oxygen uptake;
   calculating a standard exercise heart rate of the user under the standard course based on the target percentage of heart rate reserve and a maximum heart rate of the user and a resting heart rate of the user; and

calculating, by using the standard exercise heart rate and a plurality of pre-established segment model equations that reflect a relationship between heart rates of the user and exercise velocities in the standard course, the standard pace of the user in the standard course.

5. The method of claim 4, wherein the plurality of segment model equations are established by using following steps:

in a case where a continuous exercise duration of the user reaches a preset duration, dividing the continuous exercise duration into a plurality of unit time periods, wherein time periods overlap between adjacent unit time periods, and a time difference between initial time points respectively corresponding to the adjacent unit time periods is an exercise period, determining whether a historical heart rate and a historical pace within each unit time period of the plurality of unit time periods satisfy following conditions: a heart rate fluctuation within the each unit time period being within a first preset range; a velocity fluctuation within the each unit time period being within a second preset range; and a real-time percentage of heart rate reserve within the each unit time period being varying within a third preset range;
calculating an average value of historical heart rates and an average value of historical exercise velocities in each target unit time period which satisfies the conditions to use as an associated data point reflecting a relationship between the heart rates of the user and the exercise velocities, and classifying associated data points corresponding to a plurality of target unit time periods according to a plurality of predetermined segment exercise distances; and
fitting, by using associated data points corresponding to a plurality of target unit periods within each segment exercise distance of the plurality of segment exercise distances, a segment model equation corresponding to the each segment exercise distance.

6. The method of claim 2, wherein constructing the target model equation by using the pace corresponding to the maximum oxygen uptake and the real-time pace of the user in the actual exercise comprises:

constructing, by using the pace corresponding to the maximum oxygen uptake, an initial model equation reflecting the relationship between user paces and the exercise time; and
adjusting, based on the real-time pace of the user in the actual exercise, the initial model equation to obtain the target model equation.

7. The method of claim 6, wherein adjusting, based on the real-time pace of the user in the actual exercise, the initial model equation to obtain the target model equation comprises:
determining a difference between a real-time pace of the user at each exercise time point in the actual exercise and an estimated pace at the each exercise time point in the initial model equation, and adjusting the initial model equation based on the difference.

8. The method of any one of claims 1 to 7, wherein evaluating the exercise capability of the user based on the at least one user pace, wherein in the target model equation each of the at least one user pace corresponds to the exercise time point adapted in one of the at least one exercise dimension comprises:

determining, based on an exercise time point adapted in each exercise dimension of the at least one exercise dimension, a user pace in the each exercise dimension in the target model equation;
determining, based on the user pace in the each exercise dimension and a preset exercise capability score lookup table in the each exercise dimension, an exercise capability score of the user in the each exercise dimension; and
evaluating a comprehensive exercise capability of the user based on at least one exercise capability score of the user in the at least one exercise dimension.

9. An apparatus for evaluating an exercise capability, comprising:

a model invocation module, which is configured to invoke, in response to an evaluation instruction for an exercise capability of a user, a target model equation pre-constructed for the user, wherein the target model equation reflects a relationship between user paces and exercise time; and
an exercise capability evaluation module, which is configured to evaluate the exercise capability of the user based on at least one user pace, wherein in the target model equation each of the at least one user pace corresponds to an exercise time point adapted in one of at least one exercise dimension.

**10.** An electronic device, comprising:

at least one processor; and
a storage apparatus, which is configured to store at least one program;

wherein the at least one program, when executed by the at least one processor, causes the at least one processor to implement the method for evaluating an exercise capability of any one of claims 1 to 8.

**11.** A computer-readable storage medium, storing a computer program, wherein the program, when executed by a processor, implements the method for evaluating an exercise capability of any one of claims 1 to 8.

In response to an evaluation instruction for an exercise capability of a user, invoke a target model equation pre-constructed for the user , where the target model equation reflects a relationship between user paces and exercise time

S 110

Evaluate the exercise capability of the user based on at least one user pace, where in the target model equation each of the at least one user pace corresponds to an exercise time point adapted in one of at least one exercise dimension

S 120

# FIG. 1

Determine a target percentage of heart rate reserve corresponding to the maximum oxygen uptake by using a maximum oxygen uptake of the user in the standard course $\sim$ S210

Calculate a standard exercise heart rate of the user under the standard course based on the target percentage of heart rate reserve and a maximum heart rate of the user and a resting heart rate of the user $\sim$ S220

Calculate the standard pace of the user in the standard course by using the standard exercise heart rate and multiple pre-established segment model equations that reflect a relationship between heart rates of the user and exercise velocity in the standard course $\sim$ S230

Determine the pace corresponding to the maximum oxygen uptake of the user based on the standard pace of the user in the standard course $\sim$ S240

Construct an initial model equation reflecting the relationship between user paces and exercise time by using the pace corresponding to the maximum oxygen uptake $\sim$ S250

Adjust the initial model equation based on the real-time pace of the user in the actual exercise to obtain the target model equation $\sim$ S260

Evaluate the exercise capability of the user based on at least one user pace, where in the target model equation each of the at least one user pace corresponds to an exercise time point adapted in one of at least one exercise dimension $\sim$ S270

## FIG. 2

310

320

| Model invocation module | Exercise capability evaluation module |

**FIG. 3**

| Processor | 40 | Storage apparatus | 41 | Communication apparatus | 42 |

**FIG. 4**

## INTERNATIONAL SEARCH REPORT

International application No.

**PCT/CN2021/093238**

### A. CLASSIFICATION OF SUBJECT MATTER

A63B 24/00(2006.01)i

According to International Patent Classification (IPC) or to both national classification and IPC

### B. FIELDS SEARCHED

Minimum documentation searched (classification system followed by classification symbols)

A63B;G06F;G06Q

Documentation searched other than minimum documentation to the extent that such documents are included in the fields searched

Electronic data base consulted during the international search (name of data base and, where practicable, search terms used)

CNABS; DWPI; SIPOABS; CNKI; ENTXT: 运动, 能力, 体力, 评估, 速度, 配速, 时间, 模型, 方程, 乳酸, 心率, 氧; sport, capacity, body, physical, power, strength, evaluat+, speed, time, model, equation, Lactic acid, heart rate, oxygen

### C. DOCUMENTS CONSIDERED TO BE RELEVANT

| Category* | Citation of document, with indication, where appropriate, of the relevant passages | Relevant to claim No. |
|---|---|---|
| X | CN 111544853 A (GUANGDONG COROS SPORTS TECHNOLOGY CO., LTD.) 18 August 2020 (2020-08-18) claims 1-10, and description, paragraphs [0002]-[0148] | 1-2, 4-11 |
| Y | CN 111544853 A (GUANGDONG COROS SPORTS TECHNOLOGY CO., LTD.) 18 August 2020 (2020-08-18) claims 1-10, and description, paragraphs [0002]-[0148] | 3 |
| Y | CN 107595273 A (GUANGDONG YUANFENG ELECTRONIC TECHNOLOGY CO., LTD. et al.) 19 January 2018 (2018-01-19) claims 1-14, and description, paragraphs [0002]-[0175] | 3 |
| A | CN 111530037 A (GUANGDONG COROS SPORTS TECHNOLOGY CO., LTD.) 14 August 2020 (2020-08-14) entire document | 1-11 |

☐ Further documents are listed in the continuation of Box C.      ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **15 January 2022** | **27 January 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/093238**

| Patent document cited in search report | | | Publication date (day/month/year) | Patent family member(s) | | | Publication date (day/month/year) |
|---|---|---|---|---|---|---|---|
| CN | 111544853 | A | 18 August 2020 | None | | | |
| CN | 107595273 | A | 19 January 2018 | WO | 2019051969 | A1 | 21 March 2019 |
| | | | | CN | 107595273 | B | 13 December 2019 |
| CN | 111530037 | A | 14 August 2020 | CN | 111530037 | B | 01 October 2021 |

Form PCT/ISA/210 (patent family annex) (January 2015)